# EUROPÄISCHE PATENTSCHRIFT

(11) **EP 2 455 038 B1**
(45) Veröffentlichungstag und Bekanntmachung des Hinweises auf die Patenterteilung: **01.04.2015**
(21) Anmeldenummer: 12155187.3
(22) Anmeldetag: 21.10.2008
(51) Int. Cl.: A61B 19/00, A61B 17/00, A61B 19/08

(54) **Integration von chirurgischem Instrument und Anzeigevorrichtung zur Unterstützung der bildgeführten Chirurgie**
Integration of surgical instrument and display device for supporting image led surgery
Intégration d'un instrument chirurgical et dispositif d'affichage destiné à l'assistance de la chirurgie assistée par imagerie

(43) Veröffentlichungstag der Anmeldung: 23.05.2012
(62) Teilanmeldung aus: 08167095.2
(73) Patentinhaber: Brainlab AG, 85622 Feldkirchen (DE)
(72) Erfinder: Tuma, Gregor, 81677 München (DE); Neubauer, Timo, 85630 Grasbrunn-Neukeferloh (DE)
(74) Vertreter: Schwabe - Sandmair - Marx

(56) Entgegenhaltungen:
- WO-A-98/32388
- WO-A-2004/112610
- FR-A- 2 852 226
- FR-A1- 2 910 333
- US-A1- 2006 058 644
- US-A1- 2007 299 334
- US-A1- 2008 009 697
- US-A1- 2008 195 106

## Beschreibung

Die Erfindung betrifft die Integration von chirurgischen Instrumenten und Anzeigevorrichtungen (und in speziellen Ausführungsformen auch noch einer Recheneinheit) zur Unterstützung der bildgeführten Chirurgie. Insbesondere betrifft sie ein chirurgisches Instrument mit einem Griff- oder Halterungsabschnitt und einem funktionellen Abschnitt sowie ein Verfahren zum Navigieren eines chirurgischen Instruments. Bei speziellen Ausführungsformen der vorliegenden Erfindung ist das chirurgische Instrument ein Zeigeinstrument - ein so genannter Pointer.

Der genannte funktionelle Abschnitt ist derjenige Abschnitt, mit dem das Instrument gemäß seiner Aufgabe ausgestattet ist, also beispielsweise eine Zeigerspitze bei einem Pointerinstrument, eine Schneide bei einem Skalpell, ein Befestigungsabschnitt (Der Befestigungsabschnitt ist geeignet, um das Instrument woanders, insbesondere an anderen medizintechnischen Instrumenten anbringen zu können, z. B. an einem Schneidblock, einem Implantat, einer Fräse, einer Säge, einem Bohrer, einem Meißel, einem Schraubendreher usw.) oder ein zangenartiger Abschnitt bei einer Pinzette. Sie kann auch eine Kalibrierungsaufnahme sein oder umfassen, die ein Gegenstück für ein zum Instrument auszurichtendes Objekt aufweist, z.B. zum schnellen Registrieren eines Bilddatensatzes mit gescanntem Gegenstück. Diese funktionellen Abschnitte werden im täglichen Sprachgebrauch auch oft als "Spitze" des Instruments bezeichnet, und der Begriff "Spitze" wird in diesem Sinne hierin auch verwendet. Er kann also auch funktionelle Abschnitte von Instrumenten bezeichnen, die nicht körperlich als Spitze oder spitz ausgestaltet sind.

Die oben genannten Pointer werden im Rahmen der navigationsgestützten bzw. bildunterstützten Chirurgie in vielen Fällen dazu verwendet, eine Patientenregistrierung durchzuführen. Dabei werden verschiedene Punkte bzw. Landmarken am Patienten angefahren, und zwar mit einem räumlich getrackten Pointer, um die Raumlage dieser Punkte bzw. Landmarken im Navigations-Koordinatensystem eines Behandlungsraumes festzustellen. Diese Punkte oder Landmarken werden dann entsprechenden Punkten oder Landmarken zugeordnet, die in einem beispielsweise vor der Behandlung akquirierten Bilddatensatz (CT, MRI, etc.) vorhanden und im Navigationssystem abgespeichert sind. Auf dieser Basis kann dann die navigationsunterstützte bzw. bildunterstützte Behandlung ausgeführt werden. Räumlich verfolgt werden die Instrumente, also auch der Pointer, in vielen Fällen durch optische Trackingsysteme, wobei an den Instrumenten angebrachte Markierungen (Trackingmarker) von Kameras verfolgt werden.

Diese Abfolge von Registrierungsschritten ist mit getrackten Pointerinstrumenten gemäß dem Stand der Technik und herkömmlichen Navigationssystemen eher mühsam zu bewältigen, weil der Behandelnde andauernd den Blickwinkel ändern muss. Um nachzusehen, welcher Schritt als nächstes in der Registrierungsprozedur folgt, muss auf den Navigationsmonitor geblickt werden, während parallel dazu die Landmarke am Patienten gesucht wird. Um sicher zu gehen, dass die Landmarke akquiriert worden ist, muss der Chirurg wiederum auf den Monitor blicken oder ein Bestätigungssignal abwarten. Wenn mehrere Punkte oder Punktwolken (bei einer Oberflächenakquirierung) abgetastet werden müssen, wird die Situation noch schwieriger, weil die richtige Pointerstellung mit Hilfe des Monitors - gewöhnlich weit entfernt vom Arbeitsumfeld - immer wieder verifiziert werden muss.

Aus der WO 2004/112610 A2 ist eine Vorrichtung zum Erfassen und Messen von Veränderungen der Winkelposition bezüglich einer Referenzebene bekannt, die in der Chirurgie zum Ausrichten verschiedener Instrumente, Prothesen und Implantate relativ zu anatomischen Landmarken Einsatz finden kann.

Es ist eine Aufgabe der vorliegenden Erfindung, die Handhabung eines chirurgischen Instruments im Navigationsumfeld zu erleichtern, insbesondere auch den Umgang mit dem Instrument für den Chirurgen im Navigationsumfeld einfacher zu gestalten. Dies gilt speziell auch für Registrierungsaufgaben.

Diese Aufgabe wird erfindungsgemäß durch ein chirurgisches Instrument gemäß dem Anspruch 1 gelöst. Die Unteransprüche definieren bevorzugte Ausführungsformen der Erfindung.

An dem chirurgischen Instrument gemäß der vorliegenden Erfindung, das einen Griff- oder Halterungsabschnitt sowie eine Spitze aufweist, ist eine Anzeigevorrichtung vorgesehen, welche Anzeigen umfasst oder ermöglicht, die der Unterstützung der bildgeführten bzw. navigationsgestützten Chirurgie dienen. Mit anderen Worten zeigt also das Instrument selbst Informationen für den Chirurgen an, die er während des Behandlungsablaufes benötigt, so dass der Chirurg nicht den Blick vom Instrument bzw. vom Patienten nehmen muss, um navigationsgestützt arbeiten zu können. Ein Registrierungsverfahren wird beispielsweise dabei derart unterstützt, dass während der Landmarken-Akquirierungssequenz die Arbeit sehr viel intuitiver vonstatten gehen kann. Pointer oder Zeigegeräte, die erfindungsgemäß ausgestaltet sind, können nicht nur Landmarken oder Punkte positionell akquirieren, sondern ebenfalls Informationen über den Ort der Landmarke selbst zur Verfügung stellen. Der Chirurg kann seine Aufmerksamkeit auf den Behandlungsort, also das Einschnittfeld konzentrieren; er wird nicht durch Blicke zum Monitor abgelenkt.

Die Anzeigevorrichtung weist eine Bildanzeige, insbesondere eine Bildschirmanzeige auf, welche in unterschiedlichster Form ausgestaltet sein kann. Hier sind hoch auflösende Farbmonitore aber auch Strom sparende LCD-Bildanzeigen möglich. Ganz allgemein ist zu sagen, dass es im Rahmen der Erfindung wichtig ist, mit der Anzeige Informationen für den Arzt bereitzustellen, der ansonsten auf den Monitor blicken müsste. Dies kann auch Leuchtanzeigen umfassen, die punkt- oder streifenartig ausgebildet sind und beispielsweise Lastfälle mit Rot-Gelb-Grün-Anzeigen vermitteln.

Die Position des chirurgischen Instruments wird mittels eines medizintechnischen Trackingsystems bestimmt und verfolgt bzw. getrackt, wobei die Positionsdaten im Rahmen einer medizintechnischen Navigation mittels eines medizintechnischen Navigationssystems verarbeitet werden. Eine besondere Ausführungsform eines Trackingsystems ist eine eingebaute Sensorik, speziell Inertialsensorik, die zusätzlich zum optischen Trackingsystem Trackinginformation liefert.

Die Anzeige zeigt ferner Tracking-Markierungen an. Mit anderen Worten wird gemäß der Erfindung ein chirurgisches Instrument oder eine Trackingreferenz (z.B. Knochen-Tracker) bereitgestellt, dessen Anzeige-Inhalt getrackt wird. Dadurch entfällt die Notwendigkeit, Trackingmarkierungen anzubringen, aber das Tracking wird trotzdem noch robust genug sein, um das Instrument mit der Anzeigevorrichtung durchgängig und verlässlich positionell zu verfolgen. Die Anzeige kann nämlich ohne weiteres hell genug gemacht werden, um ihre Detektion durch das Kamerasystem sicherzustellen. Sie kann auch durch Farb-/Form- oder Mustergebung eindeutig genug gemacht werden.

Ferner ist der Griff- oder Halterungsabschnitt und/oder der funktionelle Abschnitt und insbesondere auch daran angebrachte Bestandteile des Instruments als sterile Wegwerfartikel, insbesondere als steril verpackter Wegwerfartikel ausgebildet, speziell aus einem Kunststoffmaterial.

Bei einer Ausführungsvariante der Erfindung ist eine Datenverarbeitung im Instrument integriert und der Anzeigevorrichtung zugeordnet, wobei mittels der Datenverarbeitung die Tätigkeit des Instruments verfolgt und identifiziert wird. Mit anderen Worten können im Instrument also durchaus auch Auswertungen der Navigations- und Trackingdaten erfolgen, die wiederum über die Anzeigevorrichtung dem Chirurgen bereitgestellt werden können.

Die Anzeigevorrichtung kann mit dem Instrument integriert vorgesehen sein oder in das Instrument integrierbar bzw. an ihm anbringbar sein, speziell über einen Adapter. Weiterhin kann die Anzeigeinformation unter Umständen direkt gematcht werden, da diese eindeutig beschreibar ist. Außerdem wird die Einheit, bei der die Trackingmarkierungen auf der Anzeigevorrichtung angezeigt werden, dazu in der Lage sein, das Tracking-Muster automatisch oder manuell zu verändern, um eine Anpassung an bestimmte Tracking-Situationen vorzunehmen oder unterschiedliche Instrumente unterschiedlichen speziellen Tracking-Mustern zuzuordnen, die dann parallel getrackt werden können.

Bei einer erfindungsgemäßen Ausführungsform umfasst die Anzeigevorrichtung die Navigations-Bildanzeige. Mit dieser erfindungsgemäßen Ausführungsform wird es für den Arzt möglich, seine gesamte Navigation bildunterstützt vorzunehmen, ohne einmal den Blick vom Arbeitsfeld, in dem sich das Instrument befindet, nehmen zu müssen.

Es ist möglich, auch die Datenverarbeitung des Navigationssystems integriert mit dem Instrument vorzusehen oder in das Instrument integrierbar bzw. an ihm anbringbar zu machen, speziell über einen Adapter. Der Arzt hätte dann ein tragbares Navigationssystem mit Anzeige und Datenverarbeitung an seinem Instrument; die Bereitstellung der üblichen Navigationskomponenten würde sich auf die Bereitstellung des Kamera-Trackingsystems beschränken. Die integrierte Datenverarbeitung kann also in solchen Fällen im Instrument die Auswertung einer Inertialsenorik, die mit im Instrument eingebauten Beschleunigungs-, Trägheits- bzw. Winkelsensoren arbeitet, übernehmen, um in Verbindung mit externer und zusätzlicher oder redundanter Trackinginformation (z.B. Kaiman Filterung) eine Verbesserung der 3D Lokalisation zu erwirken.

Eine Ausführungsform eines erfindungsgemäßen Instruments ist dadurch gekennzeichnet, dass die Datenverarbeitung und die Anzeigevorrichtung als integriertes, speziell tragbares Navigationssystem bereitgestellt sind. Dieses integrierte System, insbesondere das integrierte Navigationssystem kann am Griff oder am Halterungsabschnitt des Instruments abnehmbar über einen Adapter anbringbar sein. Die Adaption erfolgt vorzugsweise in bekannter räumlichen Zuordnung zueinander und lässt sich reproduzierbar lösen und wiederverbinden.

Das Instrument kann als Spitze eine funktionelle Instrumentenspitze haben (z.B. Pointerinstrument); es kann aber auch als Spitze ein Befestigungsabschnitt zum Fixieren des Instruments bereitgestellt werden, so dass das Instrument dann selbst ein Tracker (eine Trackingmarkierung/-referenz) wird, der beispielsweise an einem Knochen befestigt werden kann.

Das hierzu offenbarte Verfahren dient zum Navigieren eines chirurgischen Instruments, insbesondere eines Instruments, wie es oben in verschiedenen Ausführungsformen beschrieben worden ist. Die Position des Instruments wird mittels eines medizintechnischen Trackingsystems bestimmt und verfolgt bzw. getrackt, und die Positionsdaten werden im Rahmen einer medizintechnischen Navigation mittels eines medizintechnischen Navigationssystems verarbeitet, wobei Anzeigen zur Navigationsunterstützung bzw. zur Unterstützung einer bildgeführten Chirurgie am Instrument oder einem ihm positionell zugeordneten oder an ihm befestigten Element erfolgen.

Wenn mit dem Instrument, das als Positionszeiger bzw. Pointer dient, Landmarken oder Oberflächen akquiriert und registriert werden, kann die Anzeigevorrichtung die Auswahl der Landmarken bzw. Oberflächen vorgeben oder unterstützen. Dies gilt grundsätzlich auch für die Abfolge der Akquirierung.

Es ist grundsätzlich möglich, die Anzeigevorrichtung durch eine Datenverarbeitung anzusteuern, welche Bewegungen oder Punktakquirierungen mit Hilfe des Navigationssystems oder durch Sensoren am Instrument feststellt. Es ist auch möglich, Bewegungen oder Punktakquirierungen mit Hilfe einer definierten Bewegung der Tracker zueinander festzustellen.

Die bildliche Navigationsunterstützung kann über eine Anzeigevorrichtung ausgegeben werden, die als Bildschirm ausgebildet ist, und die Anzeigevorrichtung kann - wie oben schon beschrieben - wechselnde Trackingmarkierungen anzeigen. Ferner ist es möglich, dass die bildliche Navigationsunterstützung über die Anzeigevorrichtung gleichzeitig die Trackingmarkierung darstellt.

Das erfindungsgemäße Instrument kann so gestaltet werden, dass die Anzeigevorrichtung oder ein im Instrument integriertes Navigationssystem, welches die Anzeigevorrichtung umfassen kann, als separates am Instrument anbringbares Element bereitgestellt ist, das eine sterile Umhüllung, insbesondere ein steriles Drape oder eine sterile bzw. sterilisierbare Schale, aufweist.

Die Erfindung wird im Weiteren anhand der beiliegenden Zeichnungen und unter Bezugnahme auf verschiedene Ausführungsformen näher erläutert.

In den beiliegenden Zeichnungen zeigen:
- Figur 1: erfindungsgemäßer Pointer mit einem Navigationsbildschirm, der auch als Trackingreferenz dient;
- Figur 2: eine Ausführungsform der Erfindung als Knochen-Tracker,
- Figur 3: ein erfindungsgemäßes Instrument als tragbares Navigationssystem mit einer Wegwerf- oder Einmal-Halterung (sterile Lösung); und
- Figur 4: eine weitere Ausführungsform eines steril verpackten, tragbaren Navigationssystems.

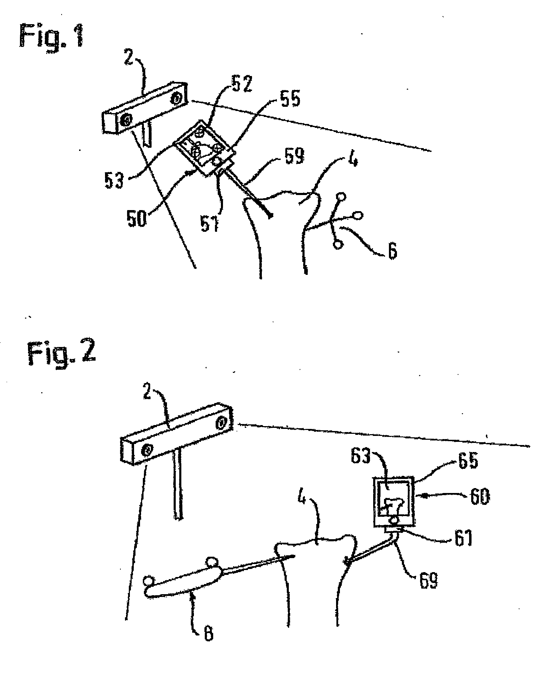

In den Figuren 1 bis 4 sind die chirurgischen Instrumente ganz allgemein jeweils mit dem Bezugszeichen 50 60 und 70 versehen worden. Die Halterungsabschnitte der Instrumente 50, 60 und 70 haben jeweils die Bezugszeichen 51, 61 und 71. Die Halterungsabschnitte 51, 61 und 71 sind als Adapterhalterungen für Anzeige- und/oder Datenverarbeitungseinheiten, insbesondere tragbare Navigationssysteme 55, 65, 75 vorgesehen, sie können aber auch feste Halterungen sein. Die Anzeigen tragen durch die Figuren jeweils die Bezugszeichen 53, 63 und 73 und Marker bzw. Trackingmarkierungen sind mit 52, 72 und 82 gekennzeichnet.

Die Instrumentenspitze 59 ist in der Figur 1 als Pointer- bzw. Zeigerspitzen ausgestaltet, während die Figur 2 eine Instrumentenspitze 69 zeigt, die an ihrem distalen Ende eine Befestigungseinrichtung aufweist. Das Bezugszeichen 52 in Figur 1 deutet auf Referenzmarker, die auf der Bildanzeige 53 erzeugt werden. In den Figuren 1 und 2 ist noch eine Trackingkamera 2 mit zwei Einzelkameras zu sehen, jeweils ein Knochen 4, in der Figur 1 eine Referenzanordnung 6 mit Referenzmarkern für den Knochen (Knochen-Tracker), und die Figur 2 zeigt noch ein weiteres Pointerinstrument 8.

Die Pointerinstrumente gemäß der vorliegenden Erfindung können nicht nur Anzeigen aufweisen, sondern auch mit einer Datenverarbeitungseinheit ausgestattet sein, die softwaregesteuert ist und die Akquirierung eines Punktes oder einer Punktewolke autonom bzw. mit Hilfe des Navigationssystems detektieren kann. Beispielsweise könnten Trägheits-Beschleunigungssensoren die momentane algorithmische Integration einer Schwenkbewegung des Pointers detektieren, um den Punkt zu akquirieren, und als Konsequenz die Anzeige auf dem Display von einem Registrierungsbildschirm zum nächsten weiterschalten.

In den Figuren 1 und 2 ist jeweils dargestellt, wie ein Navigationssystem bereitgestellt wird, das aus einer Datenverarbeitungs- und Anzeigeneinheit 55, 65 und einer extern bereitgestellten Trackingkamera 2 besteht. Es ist denkbar, dass ein externes Trackingsystem teilweise entfällt, wenn die Daten- und Anzeigeeinheit mit Inertialsensorik ausgestattet ist. Die Einheiten 55 und 65 können dabei diskrete, separate und vollständig integrierte Navigationseinheiten bzw. Anzeigeeinheiten sein. Sie sind über Adapterhalterungen 51 und 61 am Instrument befestigt. In Fällen der Figur 1 hat das Instrument eine Pointerspitze 59; im Fall der Figur 2 haltert die Spitze 69 das Instrument am Knochen 4. Durch die Navigationseinheiten 55 und 65 werden Navigationsinformationen oder Verwenderanleitungen dargestellt, und dies könnte entweder durch graphische Darstellungen oder Textinformation geschehen.

Beim Instrument gemäß Figur 1 erfolgt die Darstellung der Trackingmarker 52 direkt auf der Anzeige 53; es können also spezielle Muster erzeugt werden, die "virtuelle Marker" erzeugen. Ein Muster kann die Anzeige selbst sein, da die Anzeige zumindest in Teilaspekten durch die Programmierung der grafischen Benutzeroberfläche festgelegt ist. Die Erhöhung der Robustheit durch bekannte grafische Darstellungen kann auch dazu führen das normale Kameras verwendet werden können (statt Infrarot). Diese erfindungsgemäßen Lösungen gestatten die Vereinigung der Informations-Anzeige mit den Tracker-Einheiten innerhalb der Sichtlinie zum Trackingsystem 2. Eine zusätzliche Anbringung von Trackingmarkern ist nicht mehr notwendig.

Während das Instrument 50 der Figur 1 als Pointerinstrument zur Landmarken-Registrierung dienen kann, bei der die Patientenanatomie noch separat getrackt wird (Knochen 4 mit Trackingreferenz 6) kann gemäß der Ausführungsform nach Figur 2 das Trackingsystem selbst an dem Instrument und mit dem Instrument am Knochen 4 befestigt werden. Dadurch ist es möglich, den Knochen 4 über das Instrument 60 selbst zu tracken, und zwar über eingeblendete Marker (siehe Figur 1). Das Instrument 60 mit dem Navigations- und Anzeigesystem 65 dient also sowohl als Trackingreferenz als auch als Anzeige für den Chirurgen, und gleichzeitig kann ein weiteres Pointerinstrument 8 getrackt werden.

In allen Fällen kann der behandelnde Chirurg alle Navigationsinformationen erhalten, ohne andauernd vom Arbeitsfeld weg und auf einen separaten Navigationsmonitor blicken zu müssen.

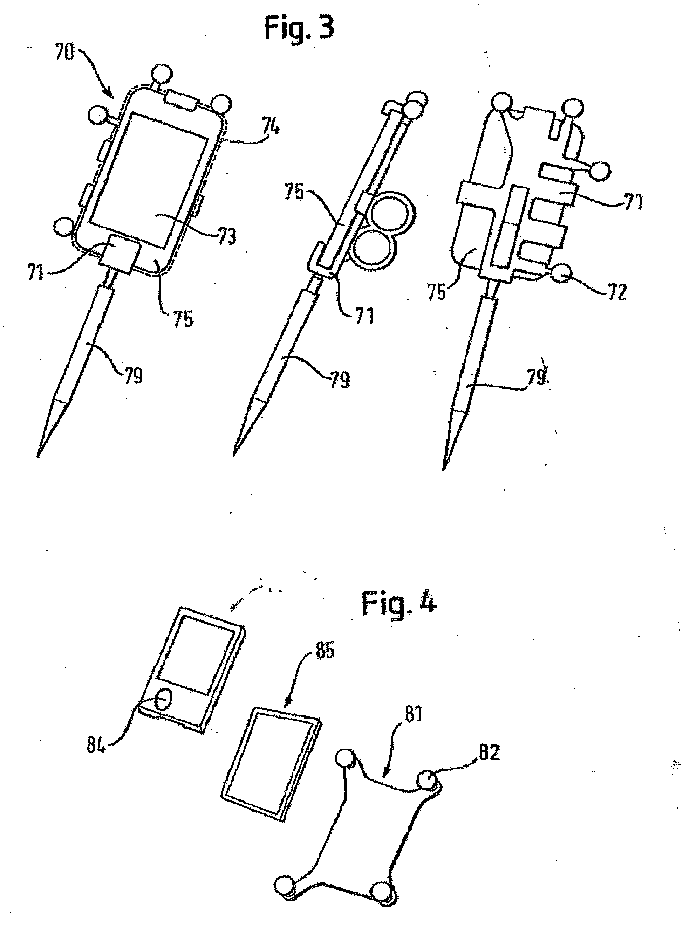

Die Figuren 3 und 4 verdeutlichen erfindungsgemäße "Sterillösungen", d.h. Ausführungsformen bei denen ein erfindungsgemäßes Instrument mit einer Anzeige oder einem daran angebrachten tragbaren Navigationssystem in einfacher und unkomplizierter Weise steril eingesetzt wird, und zwar mit Hilfe von Einweg- oder Wegwerf-Komponenten für Griff- oder Halterungsabschnitte bzw. funktionelle Abschnitte (Spitze).

Mit derartigen erfindungsgemäßen Instrumenten ist es möglich, eine Kombination eines Navigationssystems 75 (mit Anzeige 73), 85 und eines chirurgischen Instruments 70 in einer sterilen Umgebung zu verwenden. Dabei werden bei der Ausführungsform nach Figur 3 die Halteeinrichtung 71 bzw. die Spitze 79 (einzeln oder beide) als Einweg- bzw. Wegwerfartikel (einmal verwendbar) ausgebildet, die auch schon Navigations-Referenzmarker 72 tragen können. Man würde diesen Teil des Instruments einfach schon steril verpackt in der Operationsumgebung zur Verfügung stellen und nach einmaliger Verwendung entsorgen können. Er kann im Wesentlichen oder vollständig aus Kunststoff bestehen. Der Halterungsabschnitt 71 bildet einen Rahmen, in den das tragbare Navigationssystem 75 eingesetzt werden kann; er umfasst ferner Referenzmarker 72 und an ihm können alle möglichen Instrumentenspitzen angebracht sein (hier ist eine Pointerspitze 79 dargestellt). Es kann sich hierbei aber auch um eine am Halterungsabschnitt 71 angebrachte L-förmige Vorrichtung (zur Registrierung der distalen Gelenklinie und der posterioren Gelenklinie) oder um einen Schneidblock handeln.

Bei der Ausführungsform nach Figur 3 ist das tragbare Navigationssystem 75 mit der Anzeige 73 vor dem Einsetzen in die Halterung 71 in ein steriles Drape (Folienumhüllung) eingehüllt worden; dieses Drape ist gestrichelt angedeutet und hat das Bezugszeichen 74 erhalten. Auf diese Weise wird also das Navigationssystem 75 (mit Anzeige 73) ebenso steril bereitgestellt und kann unmittelbar zusammen mit den restlichen sterilen Elementen des Instruments verwendet werden. Das gesamte Instrument ist so ausgestaltet, dass der Verwender leicht mit dem Navigationssystem oder der Anzeige interagieren kann (z.B. berührungsempfindlicher Bildschirm 73), während es in der Hand gehalten und als chirurgisches Instrument verwendet wird. Wie in Figur 4 dargestellt ist, kann die Halteeinrichtung auch eine Halteplatte 81 mit Navigationsmarkern 82 aufweisen, auf der ein Navigationssystem (mit Anzeige 85) aufgebracht wird, die mit einer sterilen, starre Umhüllung (beispielsweise Abdeckung aus durchsichtigem Kunststoffmaterial) abgedeckt wird. Die Bedienung des Navigationssystems kann dann beispielsweise über dünnere Umhüllungsstellen 84 (auch im Bereich des Bildschirms möglich) erfolgen, die ein Durchtasten ermöglichen.

Insgesamt können erfindungsgemäß alle Teile des so erzeugten Instruments gemäß Figur 3 oder 4 als Einweg-Teile bereitgestellt werden, einschließlich Halterungsabschnitt, Instrumentenspitze und Referenzmarkierungen, so dass das Instrument keinen Sterilisierungsaufwand mehr erfordert. Dies gilt speziell, wenn auch die sterile Abdeckung 74 als Einweg- bzw. Wegwerfartikel bereitgestellt wird.

## Patentansprüche

1. Chirurgisches Instrument (50) mit einem Griff- oder Halterungsabschnitt (51) und einem funktionellen Abschnitt bzw. einer Spitze (59), wobei an dem Instrument eine Anzeigevorrichtung (53) vorgesehen ist, welche Anzeigen umfasst oder ermöglicht, die der Unterstützung der bildgeführten bzw. navigationsgestützten Chirurgie dienen, wobei der Griff- oder Halterungsabschnitt und/oder der funktionelle Abschnitt und insbesondere auch daran angebrachte Bestandteile des Instruments als sterile Wegwerfartikel, insbesondere als steril verpackte Wegwerfartikel ausgebildet sind, speziell aus einem Kunststoffmaterial, **dadurch gekennzeichnet, dass**
die Anzeigevorrichtung eine Bildanzeige (53) aufweist, die Tracking-Markierungen für ein Trackingsystem anzeigt.

2. Instrument nach Anspruch 1, **dadurch gekennzeichnet, dass** eine eingebaute Sensorik, speziell Inertialsensorik, Trackinginformation liefert, und die Positionsdaten im Rahmen einer medizintechnischen Navigation mittels eines medizintechnischen Navigationssystems verarbeitet werden.

3. Instrument nach einem der Ansprüche 1 bis 2, **dadurch gekennzeichnet, dass** eine Datenverarbeitung im Instrument integriert und der Anzeigevorrichtung zugeordnet ist, wobei mittels der Datenverarbeitung die Tätigkeit des Instruments verfolgt und identifiziert wird.

4. Instrument nach Anspruch 3, **dadurch gekennzeichnet, dass** die integrierte Datenverarbeitung im Instrument die Auswertung einer Inertialsenorik in Verbindung mit externer und zusätzllicher oder redundanter Trackinginformation (z.B. Kalman Filterung) übernimmt um eine Verbesserung der 3D Lokalisation zu erwirken.

5. Instrument nach einem der Ansprüche 1 bis 4, **dadurch gekennzeichnet, dass** die Anzeigevorrichtung integriert mit dem Instrument vorgesehen ist oder in das Instrument integrierbar bzw. an ihm anbringbar ist, speziell über einen Adapter ( 51, 61), wobei die Adaption in bekannter räumlichen Zuordnung zueinander von Instrument und Adapter erfolgt und reproduzierbar lösbar und wiederverbindbar ist.

6. Instrument nach einem der Ansprüche 1 bis 5, **dadurch gekennzeichnet, dass** die Anzeigevorrichtung (53, 63) die Navigations-Bildanzeige umfasst.

7. Instrument nach einem der Ansprüche 1 bis 6, **dadurch gekennzeichnet, dass** die Datenverarbeitung des Navigationssystems integriert mit dem Instrument vorgesehen ist oder in das Instrument integrierbar bzw. an ihm anbringbar ist, speziell über einen Adapter (51, 61).

8. Instrument nach einem der Ansprüche 1 bis 7, bei dem die Datenverarbeitung des Navigationssystems und die Anzeigevorrichtung integriert mit dem Instrument vorgesehen sind oder in das Instrument integrierbar bzw. an ihm anbringbar sind, **dadurch gekennzeichnet, dass** die Datenverarbeitung und die Anzeigevorrichtung als integriertes, speziell tragbares Navigationssystem bereitgestellt sind.

9. Instrument nach Anspruch 8, **dadurch gekennzeichnet, dass** das integrierte System, insbesondere das integrierte Navigationssystem am Griff oder am Halterungsabschnitt abnehmbar über einen Adapter anbringbar ist.

10. Instrument nach einem der Ansprüche 1 bis 9, **dadurch gekennzeichnet, dass** die Spitze eine funktionelle Instrumentenspitze ist, einen Befestigungsabschnitt zum Fixieren des Instruments aufweist oder eine Kalibrierungsaufnahme ist, die ein Gegenstück für ein zum Instrument auszurichtendes Objekt aufweist.

11. Instrument nach einem der Ansprüche 1 bis 10, **dadurch gekennzeichnet, dass** die Anzeigevorrichtung oder ein im Instrument integriertes Navigationssystem, welches die Anzeigevorrichtung umfassen kann, als separates am Instrument anbringbares Element bereitgestellt ist, das eine sterile Umhüllung, insbesondere ein steriles Drape oder eine sterile bzw. sterilisierbare Schale, aufweist.

## Claims

1. A surgical instrument (50) comprising a handle portion or mounting portion (51) and a functional portion and/or tip (59), wherein a display device (53) is provided on the instrument and includes or enables displays which serve to assist in image-guided and/or navigation-assisted surgery, wherein the handle portion or mounting portion and/or the functional portion and in particular also components of the instrument which are attached to them are formed as sterile disposable items, in particular as sterilely packaged disposable items, specifically made of a plastic material, **characterised in that** the display device comprises an image display (53) which displays tracking markings for a tracking system.

2. The instrument according to claim 1, **characterised in that** an integrated sensor system, specifically an inertial sensor system, provides tracking information and the position data are processed within the framework of medical navigation by means of a medical navigation system.

3. The instrument according to any one of claims 1 to 2, **characterised in that** a data processor is integrated in the instrument and assigned to the display device, wherein the activity of the instrument is tracked and identified by means of the data processor.

4. The instrument according to claim 3, **characterised in that** the integrated data processor in the instrument assumes the function of evaluating an inertial sensor system in connection with external and additional or redundant tracking information (for example, Kalman filtering), in order to improve 3D localisation.

5. The instrument according to any one of claims 1 to 4, **characterised in that** the display device is provided integrally with the instrument or can be integrated into the instrument and/or attached to the instrument, specifically via an adaptor (51, 61), wherein adapting is performed in a known spatial assignment of the instrument and the adaptor with respect to each other and can be reproducibly released and reconnected.

6. The instrument according to any one of claims 1 to 5, **characterised in that** the display device (53, 63) includes the navigation image display.

7. The instrument according to any one of claims 1 to 6, **characterised in that** the data processor of the navigation system is provided integrally with the instrument or can be integrated into the instrument and/or attached to the instrument, specifically via an adaptor (51, 61).

8. The instrument according to any one of claims 1 to 7, wherein the data processor of the navigation system and the display device are provided integrally with the instrument or can be integrated into the instrument and/or attached to the instrument, **characterised in that** the data processor and the display device are provided as an integrated, specifically portable navigation system.

9. The instrument according to claim 8, **characterised in that** the integrated system, in particular the integrated navigation system can be removably attached to the handle or to the mounting portion via an adaptor.

10. The instrument according to any one of claims 1 to 9, **characterised in that** the tip is a functional instrument tip, comprises a fastening portion for fixing the instrument, or is a calibration receptacle which comprises a counter piece for an object to be aligned to the instrument.

11. The instrument according to any one of claims 1 to 10, **characterised in that** the display device - or a navigation system which is integrated in the instrument and can include the display device - is provided as a separate element which can be attached to the instrument and comprises a sterile covering, in particular a sterile drape or a shell which is sterile and/or can be sterilised.

## Revendications

1. Instrument chirurgical (50) comportant une partie de poignée ou de fixation (51) et une partie fonctionnelle ou un embout (59), dans lequel un dispositif d'affichage (53) est prévu sur l'instrument, lequel dispositif d'affichage inclut ou permet des affichages qui servent d'aide à la chirurgie guidée par image ou assistée par navigation, dans lequel la partie de poignée ou de fixation et/ou la partie fonctionnelle et en particulier également des composants de l'instrument fixés sur celle-ci sont formés comme des articles jetables stériles, en particulier comme des articles jetables emballés de manière stérile, notamment en matière plastique, **caractérisé en ce que** le dispositif d'affichage comporte un affichage d'images (53) qui affiche des marquages de suivi pour un système de suivi.

2. Instrument selon la revendication 1, **caractérisé en ce qu'**un système de capteur intégré, en particulier un système de capteur inertiel, fournit des informations de suivi, et les données de position sont traitées dans le cadre d'une navigation médicale au moyen d'un système de navigation médical.

3. Instrument selon l'une des revendications 1 à 2, **caractérisé en ce qu'**un système de traitement de données est intégré dans l'instrument et affecté au dispositif d'affichage, dans lequel l'activité de l'instrument est suivie et identifiée au moyen du système de traitement de données.

4. Instrument selon la revendication 3, **caractérisé en ce que** le système de traitement de données intégré dans l'instrument assure l'analyse d'un système de capteur inertiel en liaison avec des informations de suivi externes et supplémentaires ou redondantes (filtrage de Kalman, par exemple) afin d'améliorer la localisation 3D.

5. Instrument selon l'une des revendications 1 à 4, **caractérisé en ce que** le dispositif d'affichage est destiné à être intégré dans l'instrument ou peut être intégré dans l'instrument ou peut être fixé sur celui-ci, en particulier par l'intermédiaire d'un adaptateur (51, 61), dans lequel l'adaptation est réalisée dans une affectation spatiale connue de l'instrument et de l'adaptateur l'un par rapport à l'autre et peut être libérée et reconnectée de manière reproductible.

6. Instrument selon l'une des revendications 1 à 5, **caractérisé en ce que** le dispositif d'affichage (53, 63) inclut l'affichage d'images de navigation.

7. Instrument selon l'une des revendications 1 à 6, **caractérisé en ce que** le système de traitement de données du système de navigation est destiné à être intégré dans l'instrument ou peut être intégré dans l'instrument ou peut être fixé sur celui-ci, en particulier par l'intermédiaire d'un adaptateur (51, 61).

8. Instrument selon l'une des revendications 1 à 7, dans lequel le système de traitement de données du système de navigation et le dispositif d'affichage sont destinés à être intégrés dans l'instrument ou peuvent être intégrés dans l'instrument ou peuvent être fixés sur celui-ci, **caractérisé en ce que** le système de traitement de données et le dispositif d'affichage sont fournis sous la forme d'un système de navigation intégré, en particulier portatif.

9. Instrument selon la revendication 8, **caractérisé en ce que** le système intégré, en particulier le système de navigation intégré, peut être fixé sur la partie de poignée ou de fixation de manière amovible, par l'intermédiaire d'un adaptateur.

10. Instrument selon l'une des revendications 1 à 9, **caractérisé en ce que** l'embout est un embout d'instrument fonctionnel, comporte une partie de fixation pour la fixation de l'instrument ou est un logement de calibrage qui inclut une pièce complémentaire pour un objet à orienter par rapport à l'instrument.

11. Instrument selon l'une des revendications 1 à 10, **caractérisé en ce que** le dispositif d'affichage ou un système de navigation intégré dans l'instrument, qui peut inclure le dispositif d'affichage, est fourni sous la forme d'un élément séparé pouvant être fixé sur l'instrument, lequel élément inclut une enveloppe stérile, en particulier un drapé stérile ou une coque stérile ou stérilisable.
